# EUROPÄISCHE PATENTSCHRIFT

(11) **EP 0 734 227 B1**
(45) Veröffentlichungstag und Bekanntmachung des Hinweises auf die Patenterteilung: **31.10.2001**
(21) Anmeldenummer: 95902710.3
(22) Anmeldetag: 14.12.1994
(51) Int. Cl.: A61B 5/15

(54) **HALTEVORRICHTUNG FÜR EIN BLUTPROBENENTNAHMERÖHRCHEN EINER BLUTABNAHMEVORRICHTUNG**
HOLDING FIXTURE FOR A BLOOD SAMPLE COLLECTION TUBE OF A BLOOD-TAKING DEVICE
SYSTEME DE FIXATION POUR TUBE DE PRELEVEMENT SANGUIN D'UN DISPOSITIF POUR PRISE DE SANG

(30) Priorität: 16.12.1993 AT 255993
(43) Veröffentlichungstag der Anmeldung: 02.10.1996
(73) Patentinhaber: Greiner Labortechnik GmbH, 4550 Kremsmünster (AT)
(72) Erfinder: KONRAD, Franz, A-4844 Regau (AT)
(74) Vertreter: Secklehner, Günter, Dr.
(86) Internationale Anmeldenummer: AT9400196
(87) Internationale Veröffentlichungsnummer: WO9516395

(56) Entgegenhaltungen:
- EP-A- 0 286 087
- EP-A- 0 323 903
- EP-A- 0 364 777
- EP-A- 0 478 459
- WO-A-89/10723
- WO-A-92/04867
- US-A- 4 819 659

## Beschreibung

Die Erfindung betrifft eine Haltevorrichtung für ein Blutprobenentnahmeröhrchen, wie sie im Oberbegriff des Patentanspruches 1 beschrieben ist.

Es sind bereits Haltevorrichtungen für Blutprobenentnahmeröhrchen einer Blutabnahmevorrichtung bekannt - gemäß WO 89/10723 A1, die einen Aufnahmebehälter für ein vorderes Ende des Blutprobenentnahmeröhrchens aufweist. Das Blutprobenentnahmeröhrchen ist an seinem vorderen Ende mit einer Verschlußvorrichtung verschlossen. Im Aufnahmebehälter ist ein Nadelhalter für eine Kanüle angeordnet, die in Richtung einer geöffneten Stirnseite des Aufnahmebehälters vorragt und deren Durchlaßöffnung über einen zwischen dem Nadelhalter und der Stirnwand des Aufnahmebehälters ausgebildeten Verbindungskanal mit einer Entnahmenadel einer Nadelanordnung verbunden ist. Diese Entnahmenadel ist exzentrisch zu einer Mittellängsachse der Aufnahmekammer angeordnet. Zwischen dem Verbindungskanal und einer Aufnahmekammer des Aufnahmebehälters ist eine bedarfsweise öffenbare Ventilanordnung vorgesehen. Diese Ventilanordnung wird durch die Kanüle und den Nadelhalter gebildet. Die Kanüle ist dazu in einer Bohrung des Nadelhalters in Längsrichtung der Mittellängsachse des Aufnahmebehälters verstellbar gelagert. Zur Begrenzung der Verstellbewegung ist die Kanüle in ihrem im Verbindungskanal angeordneten Ende mit einem über deren Außenumfang in radialer Richtung vorragenden Anschlag versehen. Dazu ist die Kanüle in ihrem dem Verbindungskanal zugewandten Ende zusammengequetscht und um 90° umgebogen und weist in der Seitenwand einen Durchbruch auf, durch den die Durchflußöffnung mit dem Verbindungskanal leitend verbunden ist. Nachteilig ist hierbei, daß durch die Nadelhalter verschiebbar gelagerte Kanüle die Gasdichtheit zwischen der Entnahmenadel und dem Blutprobenentnahmeröhrchen nur schwer erzielt werden kann und neben Undichtheiten, die teilweise auch zu einem Austreten des Blutes führen, in vielen Fällen das Vakuum der Blutprobenentnahmeröhrchen nicht zum Entnehmen von Blut ausgenutzt werden kann.

Weitere Blutentnahmevorrichtungen sind aus der US 4,819,659 A, der EP0 478 459 A1, der EP0 286 087 A2, sowie der EP0 364 777 A1 bekannt geworden, bei welchen die Kanüle jeweils durchgehend ausgebildet und in einem Nadelhalter bzw. einer Führungshülse gehaltert ist. Dabei stellt die Kanüle gleichzeitig an einem Ende die Entnahmenadel und am anderen Ende die Einstichnadel in den Verschlußstopfen des Blutprobenröhrchens dar. Zusätzlich ist die Kanüle an dem dem Blutprobenentnahmeröhrchen zugewandten Ende mit einer bedarfsweise öffenbaren Ventilanordnung in Form eines Schlauchventils umgeben. Bei diesen Ausführungsformen der Abnahmevorrichtung ist es nicht möglich, die Haltevorrichtung von der Nadelanordnung zu trennen, um beispielsweise dem Patienten eine Infusion zuführen zu können, ohne die Nadel entfernen zu müssen. Weiters besteht ein erhöhtes Sicherheitsrisiko für den Benutzer darin, daß es bedingt durch die bereits fix angeordnete Entnahmenadel schon bei der Vorbereitung der Haltevorrichtung zu Verletzungen kommen kann. Ein zusätzlicher Nachteil ergibt sich auch noch dadurch, daß die durchgängig ausgebildete Entnahmenadel zentrisch zur Haltevorrichtung angeordnet ist, wodurch bedingt durch die größere Schrägstellung der gesamten Abnahmevorrichtung beim Einstechen in die Vene ein erhöhtes zusätzliches Verletzungsrisiko besteht.

Der vorliegenden Erfindung liegt die Aufgabe zugrunde, eine Ventilanordnung für eine Blutabnahmevorrichtung im Aufnahmebehälter zu schaffen, die eine gasdichte Verbindung zwischen der Kanüle und der Entnahmenadel ermöglicht.

Diese Aufgabe der Erfindung wird durch die Merkmale im Patentanspruch 1 gelöst. Vorteilhaft ist bei dieser Ausbildung, daß durch die Verwendung einer fix in den Nadelhalter eingesetzten Kanüle nur bei entsprechender Befestigung des Nadelhalters im Aufnahmebehälter jederzeit eine definitive Verbindungs- und Dichtschicht geschaffen wird, mit welcher eine flüssigkeits- und gasdichte Verbindung zwischen der Kanüle und der Entnahmenadel einfach hergestellt werden kann. Durch den die Bundfläche überragenden und umlaufend ausgebildeten kreisringförmigen Ansatz wird durch die geringere Kreisringbreite der Verbindungsvorgang wesentlich erleichtert, da nur eine geringere Menge an Material erweicht bzw. aufgeschmolzen werden muß, um die umlaufende gas- und flüssigkeitsdichte Verbindungsschicht auszubilden. Dadurch ist auch ein Ansaugen von Fremdluft aus der Aufnahmekammer des Aufnahmebhälters in das Blutprobenentnahmeröhrchen nicht möglich. Dazu kommt, daß auch das Durchstechen der Verschlußvorrichtung im Blutprobenentnahmeröhrchen mit der Kanüle erleichtert und die Gefahr der Verletzung der Venen eines Patienten hintangehalten wird, da ruckartige Beanspruchungen, wie sie bei der bekannten Ventilanordnung bei der Bewegung der Nadel relativ zum Aufnahmebehälter bzw. beim Auftreffen der Nadel am Aufnahmebehälter aufgetreten sind, vermieden werden. Dazu kommt, daß bei Verwendung eines Schlauchventils die Durchflußöffnung der Kanüle erst nach deren Eindringen in die Verschlußvorrichtung des Blutprobenentnahmeröhrchens freigegeben wird bzw. gleichzeitig mit dem Herausziehen der Kanüle aus der Verschlußvorrichtung wieder verschlossen wird, sodaß der Austritt von Blut oder einer anderen mit dem Blutprobenentnahmeröhrchen entnommenen Körperflüssigkeit und damit nachfolgend die Gefahr einer Infektion verhindert bzw. gänzlich ausgeschaltet werden kann. Wird das Blutprobenentnahmeröhrchen betätigt, so wird der Durchfluß vom Blut bzw. der anderen Körperflüssigkeit erst nach dem Durchdringen der Verschlußvorrichtung des Blutprobenentnahmeröhrchens erst ermöglicht und andererseits ein weiterer Durchfluß des Blutes bereits unterbunden, bevor die Kanüle aus der Verschlußvorrichtung des Blutprobenentnahmeröhrchens ausgetreten ist. Ein Verschließen der Kanüle und damit verbundenen ein Austritt von Körperflüssigkeiten wird aber auch durch die Anordnung des Klappenventils im Bereich zwischen der Kanüle und dem Verbindungskanal mit Vorteil erreicht. Dabei erfolgt die Öffnungsbewegung durch das Einschieben des Entnahmeröhrchens in Verbindung mit einer Betätigungsvorrichtung, wobei nach erfolgter Abnahme das Entnahmeröhrchen mit der Verschlußvorrichtung von der Kanüle abgezogen wird und die nachströmende Körperflüssigkeit die Klappe samt der Betätigungsvorrichtung wieder in die Schließstellung verbringt. Dadurch ist wiederum ein ungewollter Austritt von Körperflüssigkeit im Anschluß an den Abnahmevorgang gesichert verhindert.

Vorteilhaft ist eine Ausführungsform nach Patentanspruch 2, da damit eine einwandfreie Positionierung des Trägerteils auf der Stirnwand des Aufnahmebehälters und damit über den gesamten Umfang die Herstellung einer gasdichten Verbindung auch bei stark wechselnden Toleranzen im Außen- und Innendurchmesser möglich ist.

Eine dichte Verbindung zwischen dem Trägerteil und dem Aufnahmebehälter wird durch die weitere Ausbildung nach Patentanspruch 3 erreicht.

Durch die Weiterbildung nach Patentanspruch 4 wird eine zusätzliche Übergangsstelle zwischen einzelnen Teilen der Blutabnahmevorrichtung verhindert und damit die Dichtheit des gesamten Systems erheblich verbessert.

Die Ausführungsvariante nach Patentanspruch 5 ermöglicht, daß trotz einer dünnen, scheibenartigen Ausbildung des Trägerteils eine massive und auch höhere Zug- und Druckkräfte aufnehmende Halterung der Kanüle im Aufnahmebehälter erreicht werden kann.

Die Ausbildungsvariante nach Patentanspruch 6 ermöglicht gleichzeitig eine einfache Herstellung des Schlauchventils, insbesondere der Befestigung der flüssigkeitsdichten Hülle desselben.

Vorteilhaft ist auch die Weiterbildung nach Patentanspruch 7, da dadurch eine einfache Befestigung der Hülle des Schlauchventils unter Verwendung des Nadelhalters erzielt werden kann.

Durch die Weiterbildung nach Patentanspruch 8 wird erreicht, daß auch bei exzentrischer, zur Mittellängsachse des Aufnahmebehälters eingesetzter Entnahmenadel ein satter, gasdichter Abschluß zwischen dem Trägerteil und dem Aufnahmebehälter erreicht werden kann.

Schließlich wird durch die Verwendung eines Kunststoffes gemäß Patentanspruch 9 neben einer ausreichenden Formstabilität auch ein fester Sitz und eine dichte Verbindung zwischen dem Trägerteil gewährleistet.

Die Erfindung wird im nachfolgenden anhand der in den Zeichnungen dargestellten Ausführungsbeispiele näher erläutert.

Es zeigen:
- Fig. 1: eine Haltevorrichtung mit einer Kanüle, einem Nadelträger und einem Blutprobenentnahmeröhrchen in Gebrauchsstellung in Seitenansicht, geschnitten, wobei der Nadelträger in seinem prinzipiellen Aufbau gezeigt wird und die erfindungsgemäßen Merkmale gemäß dem Anspruch 1 hier nicht dargestellt sind, welche jedoch in der Fig. 3 vergrößert gezeigt sind;
- Fig. 2: die Haltevorrichtung nach Fig. 1 nach erfolgter Blutabnahme bei entferntem Blutprobenentnahmeröhrchen in Seitenansicht, geschnitten;
- Fig. 3: den erfindungsgemäßen Nadelträger mit der darin gehalterten Kanüle nach den Fig. 1 und 2, in Seitenansicht, geschnitten und vergrößertem Maßstab;
- Fig. 4: eine Haltevorrichtung mit einer anderen, nicht im Schutzumfang des Anspruches 1 enthaltenen Ausführungsform des Nadelträgers, jedoch mit einer zusätzlichen Verschlußvorrichtung in Form eines Klappenventiles in der Leitungsverbindung in Seitenansicht, geschnitten und vereinfachter, schematischer Darstellung.

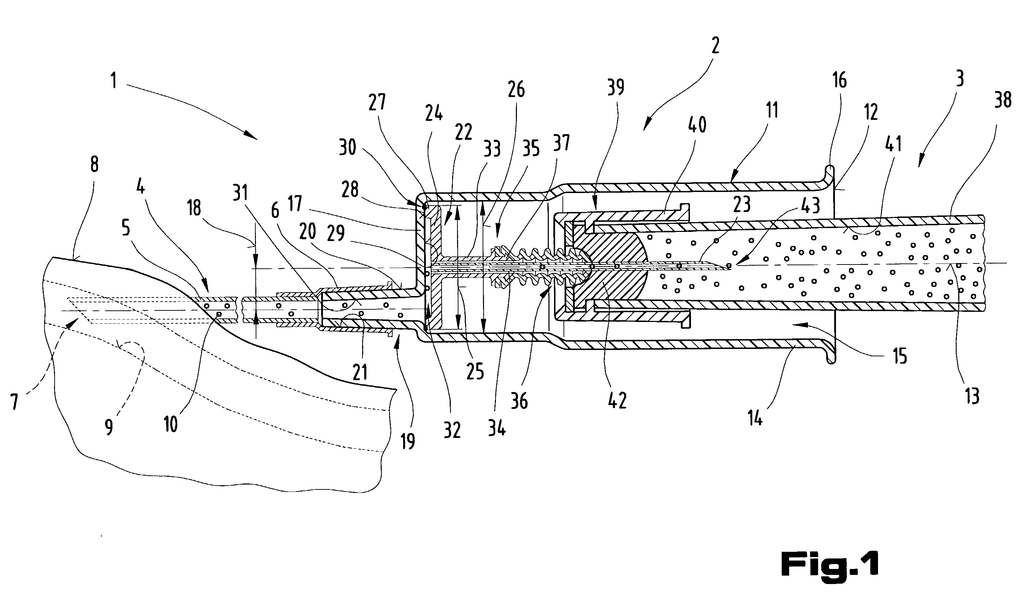

In den Fig. 1 bis 3 ist eine Blutabnahmevorrichtung 1, bestehend aus einer Haltevorrichtung 2 für ein Blutprobenentnahmeröhrchen 3 und einer mit der Haltevorrichtung 2 kuppelbaren Nadelanordnung 4 gezeigt. Die Nadelanordnung 4 besteht aus einer hohlen Entnahmenadel 5 und einem an einem Ende angeordneten Aufschubteil 6. Ein dem Aufschubteil 6 gegenüberliegendes Ende 7 der Entnahmenadel 5 weist eine Abschrägung auf und bildet eine Spitze aus, um das Einstechen in eine Haut 8 eines Patienten zu erleichtern, um in eine Vene 9 einzudringen und von dort Blut 10 - wie dies schematisch durch Kreise angedeutet ist - entnehmen zu können.

Die Haltevorrichtung 2 besteht aus einem Aufnahmebehälter 11, welcher an einer der Nadelanordnung 4 gegenüberliegenden Stirnseite 12 ein offenes Stirnende zur Aufnahme des Blutprobenentnahmeröhrchens 3 aufweist. Der Aufnahmebehälter 11 weist einen zu einer Mittellängsachse 13 konzentrisch verlaufenden Zylindermantel 14 auf und bildet somit eine zylindrische Aufnahmekammer 15 aus. Zur leichteren Bedienung der Haltevorrichtung 2 ist am Zylindermantel 14 im Bereich der Stirnseite 12 eine Handhabe 16 angeformt. Der Zylindermantel 14 ist in dem von der Stirnseite 12 abgewandten Bereich, also jenem Bereich, der der Nadelanordnung 4 zugewandt ist, mit einer senkrecht zur Mittellängsachse 13 verlaufenden Stirnwand 17 abgeschlossen.

An der Stirnwand 17 ist in einem Abstand 18 exzentrisch zur Mittellängsachse 13 ein kreisringförmiger, sich in Richtung der Nadelanordnung 4 verjüngender Halteteil 19 angeformt. Der Halteteil 19 weist an seinem Außenumfang einen sich von der Stirnwand 17 in Richtung der Nadelanordnung 4 verjüngenden Außenkonus 20 auf. Der Aufschubteil 6 der Nadelanordnung 4 weist einen gegengleich zum Außenkonus 20 ausgebildeten Innenkonus 21 auf, wodurch beim Kupplungsvorgang zwischen dem Halteteil 19 der Haltevorrichtung 2 und der Nadelanordnung 4 eine dichtende Verbindung erreicht wird. Diese Verbindung ist, wie nachfolgend noch näher erläutert werden wird, gasdicht ausgeführt.

Die Ausbildung des Halteteils 19 mit seinem Außenkonus 20 bzw. dem Aufschubteil 6 mit seinem Innenkonus 21 kann bevorzugt in Form einer Luer-Konusverbindung mit oder ohne Arretierung erfolgen. Diese Verbindungsart ist in der Medizintechnik üblicher Stand der Technik. Es kann jedoch aber selbstverständlich auch jede andere aus dem Stand der Technik bekannte Verbindungsvorrichtung zur Verbindung der Nadelanordnung 4 mit der Haltevorrichtung 2 Verwendung finden.

In der Aufnahmekammer 15 des Aufnahmebehälters 11 ist im Bereich der Stirnwand 17 ein eigener Nadelhalter 22 zur Positionierung einer Kanüle 23, welche in Form einer Hohlnadel ausgebildet ist, angeordnet. Der Nadelhalter 22 ist im vorliegenden Ausführungsbeispiel in dem der Stirnwand 17 des Haltevorrichtung 2 zugewandten Bereich durch einen scheibenförmig ausgebildeten Trägerteil 24 gebildet, welcher einen Außendurchmesser 25 aufweist, der kleiner ist als ein Innendurchmesser 26 der Aufnahmekammer 15 im Bereich der Stirnwand 17. Die Differenz zwischen diesen beiden Durchmessern wird vorteilhafterweise so groß gewählt, daß auch bei unterschiedlich auftretenden Schrumpfungen des Zylindermantels 14 der Trägerteil 24 problemlos und vor allem ohne Verklemmen in die Aufnahmekammer 15 bis zur Anlage an der Stirnwand 17 eingeschoben werden kann.

Am scheibenförmig ausgebildeten Trägerteil 24 ist ein in Richtung der Stirnwand 17 vorspringend ausgebildeter Bund 27 im Bereich des Außendurchmessers 25 mit einer im rechten Winkel zur Mittellängsachse 13 verlaufenden Bundfläche 28 des Bundes 27, welche der Nadelanordnung 4 zugewandt ist, angeordnet.

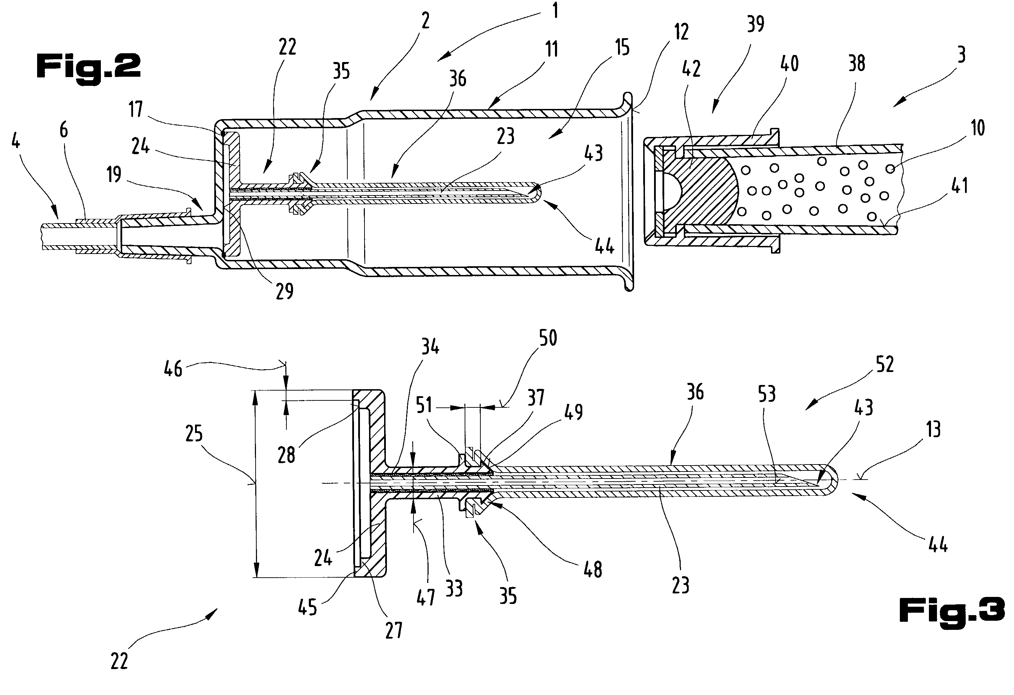

In Fig. 3 ist der Nadelhalter 22 in vergrößertem Maßstab dargestellt. Der in senkrechter Richtung zur Mittellängsachse 13 z.B. scheiben- oder kolben- bzw. plattenförmig ausgebildete Trägerteil 24 mit dem Außendurchmesser 25 weist ebenfalls den kreisringförmig ausgebildeten Bund 27 auf, welcher vorspringend an der von der Halteeinrichtung 35 abgewendeten Seite des Trägerteils 24 angeformt ist. Anschließend an die vom Bund 27 ausgebildete Bundfläche 28 ist ein kreisringförmiger Ansatz 45 mit einer geringeren Kreisringbreite 46 als die des Bundes 27 angeordnet.

Durch den umlaufenden Ansatz 45 wird eine durchgehende Anlagefläche an der Stirnwandfläche 29 des Aufnahmebehälters 11 erreicht, welche durch die geringere Kreisringbreite 46 den Verbindungsvorgang, der z.B. durch eine Hochfrequenz-Schweißung erfolgen kann, erleichtert, weil dadurch nur eine geringe Menge an Material erweicht bzw. aufgeschmolzen werden muß, um eine dichtende Anlage der Bundfläche 28 an der Stirnwandfläche 29 bevorzugt rundum durchlaufend zu erreichen.

Der Trägerteil 24 ist mittels einer bevorzugt durchgehenden in sich geschlossenen, z.B. kreisförmigen Schweißnaht 30, bevorzugt einer Hochfrequenz-Schweißnaht, gasdicht mit der Stirnwand 17 verbunden. Es ist aber selbstverständlich auch möglich, die Verbindung der beiden Bauteile mittels eines Reibschweißvorganges bzw. durch eine Kleberschichte gasdicht zu gestalten.

Der kreisringförmig ausgebildete, sich konisch verjüngende Halteteil 19 weist in seinem Inneren einen Durchflußkanal 31 auf, welcher in Strömungsverbindung mit einem Verbindungskanal 32 steht, der zwischen dem vorspringenden Bund 27 und der Stirnwand 17 verläuft.

Der Nadelhalter 22 besteht seinerseits aus einem an den Trägerteil 24 angeformten und sich in Richtung der offenen Stirnseite 12 erstreckenden kreisringförmig ausgebildeten Nadelträger 33, in welchem die Kanüle 23 bevorzugt über eine Kleberschichte 34 gehaltert ist. Sie kann jedoch auch mit dem Material des Nadelhalters 22 umspritzt sein.

Der Nadelträger 33 des Nadelhalters 22 weist an seinem der offenen Stirnseite 12 des Aufnahmebehälters 11 zugewandten Ende eine Halteeinrichtung 35 für ein strumpfartig ausgebildetes Schlauchventil 36 auf, welches durch einen elastischen, flüssigkeitsdichten Schutzüberzug, z.B. einen hochelastischen, selbstverschließenden Silikonkautschuk für die Kanüle 23 gebildet ist. Die Halteeinrichtung 35 für das Schlauchventil 36 weist eine umlaufende und über den Nadelträger 33 vorspringende und sich in Richtung der Stirnseite 12 des Aufnahmebehälters 11 konisch verjüngende Haltenase 37 auf. Die konische Verjüngung bzw. die konische Erweiterung, in Aufschubrichtung des Schlauchventils 36 gesehen, erleichtert das Überschieben hinterhalb der durch die Haltenase 37 und senkrecht zur Mittellängsachse 13 ausgerichtete über den Nadelträger 33 vorspringende Haltefläche. Das hochelastische Schlauchventil 36 zieht sich mit seinem offenen Ende hinterhalb der Haltefläche bis auf den Durchmesser des Nadelträgers 33 zusammen, wodurch das Schlauchventil 36 gegen ein unbeabsichtigtes Abziehen von der Halteeinrichtung 35 gesichert ist.

Das Blutprobenentnahmeröhrchen 3 kann, z.B. entsprechend den Angaben in der WO 89/09735, ausgebildet sein und besteht aus einem einseitig offen ausgebildeten, zylindrischen Gehäuse 38, welches an der offenen Stirnseite mittels einer gasdichten Verschlußvorrichtung 39 verschlossen ist. Die Verschlußvorrichtung 39 besteht ihrerseits wiederum aus einer das Gehäuse 38 übergreifenden Kappe 40 und einem einen Innenraum 41 des Gehäuses 38 verschließenden Stopfen 42. Der mit dem Stopfen 42 verschlossene Innenraum 41 des Blutprobenentnahmeröhrchens 3 ist bei ungebrauchtem Zustand, z.B. auf ein Vakuum zwischen 10 kPa und 80 kPa, evakuiert.

Um ein leichtes und sicheres Durchstechen der Kanüle 23 durch den Stopfen 42 des Blutprobenentnahmeröhrchens 3 sicherzustellen, weist die Kanüle 23 an dem der Stirnseite 12 des Aufnahmebehälters 11 zugewandten Ende eine Abschrägung auf, welche somit eine Spitze 43 ausbildet.

Bei der in Fig. 1 dargestellten Gebrauchsstellung der Blutabnahmevorrichtung 1 besteht nun eine Leitungsverbindung zwischen der Vene 9 und dem evakuierten Innenraum 41 des Blutprobenentnahmeröhrchens 3. Diese Leitungsverbindung ist gebildet durch die hohle Entnahmenadel 5, den daran anschließenden Durchflußkanal 31 des Halteteils 19, den Verbindungskanal 32 zwischen dem Trägerteil 24 und der Stirnwand 17 und der nachfolgenden, hohl ausgebildeten Kanüle 23, welche im Nadelhalter 22 gehaltert ist und deren Durchlaßöffnung in den Innenraum 41 des Blutprobenentnahmeröhrchens 3 mündet. Der im Blutprobenentnahmeröhrchen 3 herrschende Unterdruck dient dazu, die in der Leitungsverbindung auftretenden Strömungsverluste bzw. Fließwiderstände zu überbrücken, da sonst der in den Venen herrschende Druck nicht ausreicht, das zu entnehmende Blut 10 bis in das Blutprobenentnahmeröhrchen 3 zu drücken.

Bei der hier dargestellten Ausführungsform des Blutprobenentnahmeröhrchens 3, welches im ungebrauchten Zustand im Innenraum 41 ein Vakuum aufweist, ist entscheidend, daß die zuvor beschriebenen Bauteile, welche die Leitungsverbindung zwischen der Vene 9 und dem Innenraum des Blutprobenentnahmeröhrchens 3 bilden, gasdicht ausgebildet sind, um so ein einwandfreies Ansaugen von Blut 10 aus der Vene 9 zu garantieren. Der von der Kanüle 23 zu durchstoßende Stopfen 42 des Blutprobenentnahmeröhrchens 3 ist aus einem hochelastischen und selbstverschließenden Werkstoff, bevorzugt Pharmagummi oder Silikonkautschuk, hergestellt, um nach dem Entfernen des Blutprobenentnahmeröhrchens 3 von der Haltevorrichtung 2 den Innenraum 41 wieder gesichert abzudichten.

In der Fig. 2 ist die Blutabnahmevorrichtung 1 in einer Stellung gezeigt, bei welcher das Blutprobenentnahmeröhrchen 3 von der in Fig. 1 dargestellten Stellung von der Kanüle 23 distanziert angeordnet ist. Dabei ist der Innenraum 41 des Blutprobenentnahmeröhrchens 3 durch den selbsttätig verschließenden Stopfen 42 gegenüber der Außenumgebung abgedichtet und mit schematisch durch Kreise angedeutetem Blut 10 gefüllt.

Das hochelastisch ausgebildete Schlauchventil 36 ist von der in Fig. 1 gezeigten zusammengeschobenen Stellung in Richtung der Stirnseite 12 des Aufnahmebehälters 11 expandiert und ein Ende 44 des elastischen Schutzüberzuges umschließt die Spitze 43 der Kanüle 23. Dadurch, daß der Werkstoff des Schlauchventils 36 hochelastisch gewählt wurde und hohe Rückstellwerte aufweist, verschließt sich der Durchstich der Spitze 43 der Kanüle 23 im Ende 44 selbsttätig. Dadurch ist ein dichtender Abschluß in Form einer Ventilwirkung an dem der Stirnseite 12 zugewandten Ende der Kanüle 23 erreicht.

Soll nun eine erneute Blutabnahme erwünscht sein, muß lediglich ein neues Blutprobenentnahmeröhrchen 3 mit seinem evakuierten Innenraum 41 erneut in die Aufnahmekammer 15 des Aufnahmebehälters 11 eingeschoben werden, bis wiederum eine Anlage des Stopfens 42 am Ende 44 des Schlauchventils 36 erreicht ist. Bei einem weiteren Einschieben des Blutprobenentnahmeröhrchens 3 in Richtung der Stirnwand 17 durchsticht die Spitze 43 der Kanüle 23 das Ende 44 des Schlauchventils 36 und dringt durch den elastischen Stopfen 42 in den Innenraum 41 des Blutprobenentnahmeröhrchens 3 ein. Somit ist wiederum eine nach außen hin dichtend abgeschlossene Leitungsverbindung zwischen der Vene 9 und dem Innenraum 41 des Blutprobenentnahmeröhrchens 3 sichergestellt.

Bei dem in der Fig. 3 gezeigten Nadelhalter 22 ist die Kanüle 23 dichtend mittels der Kleberschichte 34 im Nadelträger 33 des Nadelhalters 22 eingeklebt. Es ist aber auch möglich, die Kanüle 23 beim Formvorgang des Nadelhalters 22 mit diesem einstückig zu verbinden oder einen über die übliche Verwendungstemperatur vorgewärmten Nadelträger und/oder eine abgekühlte Kanüle ineinander einzusetzen und anschließend auf Raumtemperatur zu bringen, wodurch ebenfalls ein dichtender Schrumpfsitz zwischen Kanüle 23 und Nadelhalter 22 erzielbar ist.

Wie weiters besser aus der vergrößerten Darstellung der Halteeinrichtung 35 zu ersehen ist, ragt die Haltenase 37 mit ihrem dem Trägerteil 24 zugewandten Ende über einen Durchmesser 47 des Nadelträgers 33 vor. Die rundum laufende und vorspringende Haltenase 37 ist als sich in Richtung der Spitze 43 der Kanüle 23 verjüngender Kegelstumpf 48 ausgebildet und dient dem Schlauchventil 36 als Haltekonus 49. Die Halteeinrichtung 35 weist weiters zur besseren Lagefixierung des Schlauchventils 36 einen um eine Distanz 50 von der Haltenase 37 in Richtung des Trägerteils 24 distanzierten flanschförmigen Ansatz 51 auf, welcher ebenfalls den Durchmesser 47 des Nadelträgers 33 in radialer Richtung, also senkrecht zur Mittellängsachse 13 überragt.

Das über die Kanüle 23 reichende und an der Halteeinrichtung 35 gehalterte Schlauchventil 36 bildet eine dichtende Ventilanordnung 52 aus. Dadurch umschließt bei abgezogenem Blutprobenentnahmeröhrchen 3 das Ende 44 des Schlauchventils 36 die Spitze 43 der Kanüle 23 und dichtet somit eine Durchflußöffnung 53 der Kanüle 23 gegenüber der Aufnahmekammer 15 des Aufnahmebehälters 11 ab. Dadurch ist die zuvor bereits beschriebene Leitungsverbindung zwischen dem Ende 7 der Nadelanordnung 4 und der Spitze 43, welche der offenen Stirnseite 12 des Aufnahmebehälters 11 zugewandt ist, der Kanüle 23 dichtend abgeschlossen.

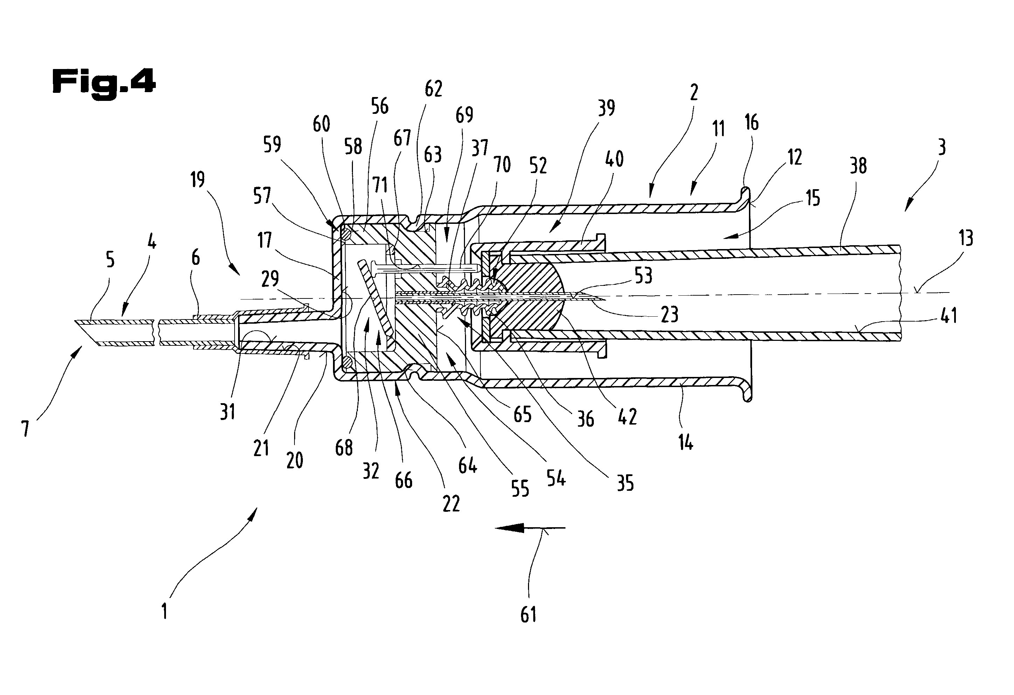

In Fig. 4 ist eine andere Blutabnahmevorrichtung 1 mit der Haltevorrichtung 2 und dem Blutprobenentnahmeröhrchen 3 gezeigt, wobei für gleiche Teile gleiche Bezugszeichen verwendet wurden. Hierbei ist eine andere Ausführung der Ventilanordnung gezeigt, welche durch ein in der Leitungsverbindung angeordnetes Klappenventil gebildet ist. Die hier gezeigte und beschriebene Ausbildung des Nadelhalters 22, dessen Halterung im Aufnahmebehälter 11 sowie die Abdichtung zwischen diesen Bauteilen ist nicht Gegenstand der Erfindung.

Das Blutprobenentnahmeröhrchen 3 besteht aus dem einseitig verschlossenen Gehäuse 38, der Verschlußvorrichtung 39, bestehend aus der Kappe 40 und den den Innenraum 41 dichtend verschließenden Stopfen 42.

Die Haltevorrichtung 2 für die Kanüle 23 ist gebildet durch den einseitig mit der Stirnwand 17 verschlossenen Aufnahmebehälter 11, den exzentrisch zur Mittellängsachse 13 an der Stirnwand 17 angeformten Halteteil 19 mit seinem Außenkonus 20 und der an der offenen Stirnseite 12 angeordneten Handhabe 16. Am Halteteil 19 ist wiederum die Nadelanordnung 4, bestehend aus der hohlen Entnahmenadel 5 und dem Aufschubteil 6 mit seinem Innenkonus 21, gehaltert.

Der Nadelhalter 22 für die Kanüle 23 weicht von der zuvor beschriebenen Ausführungsform ab und ist als topfförmiger Trägerteil 54 ausgebildet und besteht aus einer scheibenförmigen Haltewand 55, an welche ein kreisringförmig ausgebildeter Vorsprung 56 angeformt ist.

An einer der Stirnwandfläche 29 der Stirnwand 17 zugewandten Aufnahmefläche 57 ist eine halbkreisförmig ausgebildete, rundum laufende Ausnehmung 58 eingeformt, welche zur Aufnahme eines Dichtelementes 59, wie z.B. eines O-Rings 60, dient. Bedingt durch das elastisch verformbare Dichtelement 59 und den durch den Vorsprung 56 gebildeten Hohlraum entsteht wiederum der in Strömungsverbindung mit dem Durchflußkanal 31 stehende Verbindungskanal 32, wodurch ebenfalls eine dichtende Verbindung zwischen dem Nadelhalter 22 und dem Aufnahmebehälter 11 gewährleistet ist.

Um eine exakte Positionierung bzw. Halterung des Trägerteils 54 im Aufnahmebehälter 11 sicherzustellen bzw. einen dichtenden Übergang zwischen der Stirnwandfläche 29 und der Aufnahmefläche 57 herzustellen, wird der Nadelhalter 22 in Richtung eines Pfeils 61 gegen die Stirnwandfläche 29 gedrückt, wodurch sich das Dichtelement 59 verformt und anschließend wird in dieser Position der Zylindermantel 14 thermisch eingedrückt bzw. verformt und ein so entstehender Wulst 62 ragt in eine am Außenumfang der Haltewand 55 eingeformte Haltenut 63 hinein. Dieser Wulst 62 stützt sich an einer sich konisch in Richtung der Aufnahmefläche 57 erweiterten Haltefläche 64 ab, wodurch das vorgespannte Dichtelement 59 einerseits den Verbindungskanal 32 nach außen hin abdichtet und andererseits die Haltefläche 64 gegen den Wulst 62 preßt und so eine gesicherte Lagefixierung darstellt.

An einer der Stirnseite 12 des Aufnahmebehälters 11 zugewandten Stirnfläche 65 des Nadelhalters 22 ist wiederum die Halteeinrichtung 35 für das Schlauchventil 36 der Ventilanordnung 52 angeordnet. Die Halteeinrichtung 35 besteht wiederum aus der zuvor bereits beschriebenen Haltenase 37, welche kegelstumpfförmig ausgebildet ist. Den Längsanschlag für das Schlauchventil 36 in Richtung der Stirnwand 17 bildet bei diesem Ausführungsbeispiel die Stirnfläche 65 aus.

Zusätzlich zur Ventilanordnung 52 ist in dem durch den Vorsprung 56 gebildeten Verbindungskanal 32 eine zusätzliche Verschlußvorrichtung 66 angeordnet. Diese besteht aus einem Einsatzteil 67, welcher im Verbindungskanal 32 dichtend eingesetzt ist und einer mit dieser einstückig und gelenkig verbundenen Klappe 68, welche die Durchflußöffnung 53 der Kanüle 23 gegenüber dem Verbindungkanal 32 abschließt. Die Verstellung der Klappe erfolgt durch eine Betätigungsvorrichtung 69, welche durch eine Schubstange 70 gebildet ist.

Es ist aber selbstverständlich auch möglich, entweder die Ventilanordnung 52 durch ein zwischen der Kanüle 23 und der Aufnahmekammer 15 angeordnetes Schlauchventil 36 oder durch ein zwischen der Kanüle 23 und dem Verbindungskanal 32 angeordnetes Klappenventil auszubilden.

Bei der in Fig. 4 dargestellten Gebrauchslage der Blutabnahmevorrichtung 1 liegt ein Teil der Verschlußvorrichtung 39 des Blutprobenentnahmeröhrchens 3 an dem der Stirnseite 12 zugewandten Ende der Schubstange 70 an, wodurch diese in einer in der Haltewand 55 angeordneten Öffnung 71 in Richtung der Mittellängsachse 13 verschoben wird und bei weiterem Einschieben des Blutprobenentnahmeröhrchens 3 in die Aufnahmekammer 15 jenes Ende, welches der Stirnwand 17 zugeordnet ist, die Klappe 68 in die gezeigte geöffnete Stellung bringt. Wird das Blutprobenentnahmeröhrchen 3 aus der Aufnahmekammer 15 nach erfolgter Blutabnahme entfernt, so drückt das in der Nadelanordnung 4 befindliche Blut die Klappe 68 samt der Schubstange 70 wieder in eine geschlossene Stellung, wodurch die Leitungsverbindung zwischen dem Verbindungskanal 32 und der Durchflußöffnung 53 in der Kanüle 23 unterbrochen ist.

Weiters ist es vorteilhaft, wenn zwischen dem Halteteil 19 und einem diesem nächstliegenden Bereich des Zylindermantels 14 eine im wesentlichen senkrecht zur Mittellängsachse 13 verlaufende Auflagefläche für den Trägerteil 24 angeordnet ist. Durch diese Ausbildung wird auch bei exzentrischer, zur Mittellängsachse 13 des Aufnahmebehälters 11 eingesetzter Entnahmenadel ein satter und gasdichter Abschluß zwischen dem Trägerteil 24 und dem Aufnahmebehälter 11 erreicht.

Außerdem ist es möglich, den Kunststoff über Lösungsmittel aufzuweichen, sodaß es unter Ausnutzung der dem Kunststoff innewohnenden Klebewirkung möglich ist, diese Teile miteinander, insbesondere gasdicht zu verbinden. Als vorteilhaft hat sich erwiesen, wenn der Nadelhalter 22 bzw. der Trägerteil 24 und/oder der Aufnahmebehälter 11 aus Kunststoff, insbesondere Polypropylen bestehen.

Um diese Gasdichtheit durchgängig von der Nadelanordnung 4 bis hin zum Innenraum 41 des Blutprobenentnahmeröhrchens 3 zu erreichen, muß gewährleistet sein, daß sowohl alle Bauteile als auch die einzelnen Schweiß- bzw. Verbindungsstellen zwischen diesen Bauteilen entsprechende Eigenschaften aufweisen. Dies ist auch bei der Wahl der einzelnen Werkstoffe zu berücksichtigen.

Abschließend sei darauf hingewiesen, daß bei der Darstellung der Ausführungsbeispiele zum besseren Verständnis der Funktion der erfindungsgemäßen Lösungen und zur vereinfachten Darstellung Proportionen verzerrt und einzelne unmaßstäblich dargestellt sind.

## Patentansprüche

1. Haltevorrichtung (2) für ein Blutprobenentnahmeröhrchen (3) einer Blutabnahmevorrichtung (1) mit einem Aufnahmebehälter (11) für ein durch eine Verschlußvorrichtung (39) verschlossenes vorderes Ende des Blutprobenentnahmeröhrchens (3), mit einem einen Trägerteil (24) umfassenden Nadelhalter (22), in dem eine zur geöffneten Stirnseite (12) des Aufnahmebehälters (11) gerichtete Kanüle (23) gehaltert ist und in Richtung derselben vorragt und der Nadelhalter (22) konzentrisch zum Aufnahmebehälter (11) angeordnet ist und der Trägerteil (24) einen in die von der Kanüle (23) abgewendete Richtung vorragenden, kreisringförmigen Bund (27) mit einer Bundfläche (28) aufweist und eine Durchflußöffnung der Kanüle (23) über einen zwischen dem Nadelhalter (22) und einer Stirnwand (17) des Aufnahmebehälters (11) ausgebildeten Verbindungskanal (32) mit einer exzentrisch zu einer Mittellängsachse (13) einer Aufnahmekammer (15) des Aufnahmebehälters (11) angeordneten Entnahmenadel (5) einer Nadelanordnung (4) verbunden ist und mit einer zwischen dem Verbindungskanal (32) und der Aufnahmekammer (15) des Aufnahmebehälters (11) angeordneten, bedarfsweise öffenbaren Ventilanordnung (52), welche durch ein zwischen der Kanüle (23) und der Aufnahmekammer (15) angeordnetes Schlauchventil (36) oder durch ein Klappenventil zwischen der Kanüle (23) und dem Verbindungskanal (32) gebildet ist, **dadurch gekennzeichnet, daß** die Kanüle (23) gas- und flüssigkeitsdicht im Nadelhalter (22) bewegungsfest gehaltert ist und an der Bundfläche (28) des Bundes (27) ein umlaufender kreisringförmiger Ansatz (45) mit einer geringeren Kreisringbreite (46) als die des Bundes (27) angeordnet ist und daß der Trägerteil (24) des Nadelhalters (22) im Bereich des Ansatzes (45) über eine umlaufende gas- und flüssigkeitsdichte Verbindungsschicht, insbesondere eine Schweißnaht (30) oder eine Kleberschichte, mit dem Aufnahmebehälter (11), insbesondere dessen Stirnwand (17), verbunden ist.

2. Haltevorrichtung nach Anspruch 1, **dadurch gekennzeichnet, daß** der Trägerteil (24) des Nadelhalters (22) einen kleineren Außendurchmesser (25) aufweist als ein Innendurchmesser (26) des Aufnahmebehälters (11) im Bereich des Trägerteils (24).

3. Haltevorrichtung nach Anspruch 1 oder 2, **dadurch gekennzeichnet, daß** der Trägerteil (24) scheibenartig ausgebildet ist.

4. Haltevorrichtung nach Anspruch 1, **dadurch gekennzeichnet, daß** ein Durchflußkanal (31) eines Halteteils (19) zwischen dem umlaufenden Bund (27) und der Kanüle (23) in den Verbindungskanal (32) mündet.

5. Haltevorrichtung nach einem oder mehreren der Ansprüche 1 bis 3, **dadurch gekennzeichnet, daß** an dem Trägerteil (24) ein sich in Richtung der offenen Stirnseite (12) des Aufnahmebehälters (11) erstreckender Nadelträger (33) angeordnet ist, in dem die Kanüle (23) mit einem Teilbereich Ihrer Länge hineinragt.

6. Haltevorrichtung nach Anspruch 5, **dadurch gekennzeichnet, daß** an dem der Kanüle (23) zugewandten Stirnende des Nadelträgers (33) eine Halteeinrichtung (35) für das Schlauchventil (36), insbesondere eine strumpfförmige, flüssigkeitsdichte Hülle (74) angeordnet ist.

7. Haltevorrichtung nach Anspruch 6, **dadurch gekennzeichnet, daß** der Nadelhalter (22) als Halteeinrichtung (35) für die Hülle (74) des Schlauchventils (36) als ein sich in eine vom Trägerteil (24) abgewendete Richtung kegelförmig verjüngender Haltekonus (49), insbesondere als Kegelstumpf (48) ausgebildet ist.

8. Haltevorrichtung nach Anspruch 1, **dadurch gekennzeichnet, daß** zwischen dem Durchflußkanal (31) des Halteteils (19) und einem diesem nächstliegenden Bereich des Zylindermantels (14) eine im wesentlichen senkrecht zur Mittellängsachse (13) verlaufende Auflagefläche für den Trägerteil (24) angeordnet ist.

9. Haltevorrichtung nach Anspruch 1, **dadurch gekennzeichnet, daß** der Trägerteil (24) und/oder der Aufnahmebehälter (11) aus Kunststoff, insbesondere Polypropylen, besteht.

## Claims

1. Holding fixture (2) for a blood sample collection tube (3) of a blood taking device (1), having a housing container (11) for a front end of a blood sample collection tube (3) closed off by a closure device (39) incorporating a needle holder (22) with a holder part (24) in which a cannula (23) directed towards the open- end face (12) of the housing container (11) is retained and projecting in the direction thereof, the needle holder (22) being disposed concentrically with the housing container (11) and the holder part (24) having an annular collar (27) with a collar face (28) projecting in the direction remote from the cannula (23), and a passage orifice of the cannula (23) is linked via a connecting passage (32) between the needle holder (22) and the end wall (17) of the housing container (11) to an intake needle (5) of a needle arrangement (4) disposed eccentrically relative to a central longitudinal axis (13) of a housing chamber (15) of the housing container (11) and having a valve arrangement (52) disposed between the connecting passage (32) and the housing chamber (15) of the housing container (11) which can be opened as necessary, provided in the form of a pipe compression valve (36) arranged between the cannula (23) and the housing chamber (15) or a flap valve between the cannula (23) and the connecting passage (32), **characterised in that** the cannula (23) is held stationary in the needle holder (22), being gas- and fluid-tight, and a circumferential annular shoulder (45) of a smaller annular width (46) than the collar (27) is provided on the collar face (28) of the collar (27) and, in the region of the shoulder (45), the holder part (24) of the needle holder (22) is joined to the housing container (11), in particular the end wall (17) thereof, by means of a circumferential gas- and fluid-tight joining layer, in particular a weld seam (30) or a bond seam.

2. Holding fixture as claimed in claim 1, **characterised in that** the holder part (24) of the needle holder (22) has a smaller external diameter (25) than an internal diameter (26) of the housing container (11) in the region of the holder part (24).

3. Holding fixture as claimed in claim 1 or 2, **characterised in that** the holder part (24) is of a disc-type design.

4. Holding fixture as claimed in claim 1, **characterised in that** a flow passage (31) of a retaining part (19) opens into the connecting passage (32) between the circumferential collar (27) and the cannula (23).

5. Holding fixture as claimed in one or more of claims 1 to 3, **characterised in that** a needle holder (33) extending in the direction towards the open end face (12) of the housing container (11) is provided on the holder part (24), into which the cannula (23) projects by a part region of its length.

6. Holding fixture as claimed in claim 5, **characterised in that** a holding device (35) for the pipe compression valve (36), in particular a hose-shaped fluid-tight cover (74), is provided on the end face of the needle holder (33) facing the cannula (23).

7. Holding fixture as claimed in claim 6, **characterised in that**, being a holding device (35) for the cover (74) of the pipe compression valve (36), the needle holder (22) is provided in the form of a retaining cone (49) conically tapered in a direction remote from the holder part (24), in particular a truncated cone (48).

8. Holding fixture as claimed in claim 1, **characterised in that** a bearing surface for the holder part (24), substantially perpendicular to the central longitudinal axis (13), is provided between the flow passage (31) of the retaining part (19) and a region of the cylinder jacket (14) lying adjacent thereto.

9. Holding fixture as claimed in claim 1, **characterised in that** the holder part (24) and/or the housing container (11) is/are made from plastics, in particular polypropylene.

## Revendications

1. Dispositif de retenue (2) pour un tube de prélèvement sanguin (3) d'un dispositif pour prise de sang (1) avec un récipient de collecte (11) pour une extrémité avant du tube de prélèvement sanguin (3) fermée par un dispositif de fermeture (39), avec un porte-aiguille (22) comprenant une partie de support (24) dans lequel est retenue une canule (23) orientée vers le côté frontal ouvert (12) du récipient de collecte (11) et fait saillie dans la direction de celle-ci, et le porte-aiguille (22) est disposé d'une manière concentrique au récipient de collecte (11), et la partie de support (24) présente un épaulement (27) en anneau de cercle, faisant saillie dans la direction éloignée de la canule (23), avec une face d'épaulement (28), et une ouverture d'écoulement de la canule (23) est reliée par un canal de liaison (32) réalisé entre le porte-aiguille (22) et une paroi frontale (17) du récipient de collecte (11) à une aiguille de prélèvement (5) d'un agencement d'aiguille (4) disposé d'une manière excentrique à un axe longitudinal médian (13) d'une chambre de réception (15) du récipient de collecte (11), et avec un agencement de vanne (52) disposé entre le canal de liaison (32) et la chambre de réception (15) du récipient de collecte (11), pouvant être ouvert en cas de besoin, qui est formé par une vanne à gaine (36) disposée entre la canule (23) et la chambre de réception (15) ou par une vanne à clapet entre la canule (23) et le canal de liaison (32), **caractérisé en ce que** la canule (23) est retenue fixement, d'une manière étanche au gaz et au liquide dans le porte-aiguille (22) et qu'il est disposé à la face d'épaulement (28) de l'épaulement (27) un bout rapporté (45) en forme d'anneau de cercle, s'étendant tout autour, avec une plus petite largeur d'anneau de cercle (46) que celle de l'épaulement (27) et **en ce que** la partie de support (24) du porte-aiguille (22) est reliée au voisinage du bout rapporté (45) par une couche de liaison étanche au gaz et au liquide s'étendant tout autour, notamment une soudure (30) ou une couche de colle, au récipient de collecte (11), notamment à sa paroi frontale (17).

2. Dispositif de retenue selon la revendication 1,
**caractérisé en ce que** la partie de support (24) du porte-aiguille (22) présente un plus petit diamètre extérieur (25) qu'un diamètre intérieur (26) du récipient de collecte (11) au voisinage de la partie de support (24).

3. Dispositif de retenue selon la revendication 1 ou 2,
**caractérisé en ce que** la partie de support (24) est réalisée en forme de disque.

4. Dispositif de retenue selon la revendication 1,
**caractérisé en ce qu'**un canal d'écoulement (31) d'une partie de retenue (19) entre l'épaulement s'étendant tout autour (27) et la canule (23) débouche dans le canal de liaison (32).

5. Dispositif de retenue selon l'une ou plusieurs des revendications 1 à 3, **caractérisé en ce qu'**il est disposé à la partie de support (24) un porte-aiguille (33) s'étendant dans la direction du côté frontal ouvert (12) du récipient de collecte (11) dans lequel fait saillie la canule (23) avec une zone partielle de sa longueur.

6. Dispositif de retenue selon la revendication 5,
**caractérisé en ce qu'**il est disposé au côté frontal du porte-aiguille (33) orienté vers la canule (23) une installation de retenue (35) pour la vanne à gaine (36), notamment une gaine (74) en forme de bas, étanche au liquide.

7. Dispositif de retenue selon la revendication 6,
**caractérisé en ce que** le porte-aiguille (22) est réalisé comme installation de retenue (35) pour la gaine (74) de la vanne à gaine (36) comme un cône de retenue (49), notamment comme cône tronqué (48) diminuant en forme de cône dans une direction éloignée de la partie de support (24).

8. Dispositif de retenue selon la revendication 1,
**caractérisé en ce qu'**il est disposé entre le canal d'écoulement (31) de la partie de retenue (19) et une zone de l'enveloppe cylindrique (14) la plus proche de celle-ci une face d'application s'étendant sensiblement perpendiculairement à l'axe longitudinal médian (13) pour la partie de support (24).

9. Dispositif de retenue selon la revendication 1,
**caractérisé en ce que** la partie de support (24) et/ou le récipient de collecte (11) est en matière synthétique, notamment en polypropylène.
